# EUROPEAN PATENT APPLICATION

(11) **EP 4 316 562 A1**
(43) Date of publication of application: **07.02.2024**
(21) Application number: 22460058.5
(22) Date of filing: 26.10.2022
(51) Int. Cl.: A61M 25/01, A61M 25/00

(54) **ENDOVASCULAR CATHETER**

(30) Priority: 05.08.2022 PL 44194322
(71) Applicant: Sniper sp. z o.o., 91-134 Lodz (PL)
(72) Inventor: MIZERA, Ireneusz, 98-220 Zdunska Wola (PL)
(74) Representative: Dziubinska, Joanna

(57) **Abstract**

Endovascular catheter is characterized in that has a two-element structure, consisting of two thin tubes (1) and (2) of different diameters, the inner tube (2) being thinner than the outer tube (1) and placed inside a wider outer tube (1), both tubes (1) and (2) having curved ends (3) in the distal part, whose bend radius differs; wherein the bend radius of the ends (3) of both tubes (1) and (2) in the distal section is between 45-90°; the curved ends (3) rotate independently of each other around a common axis and longitudinally slide one tube into the other.

## Description

The subject of the invention is a new and innovative endovascular catheter intended for use in vascular surgeries performed according to intravascular methods. Various types of endovascular catheters are known.

Patent No. PL234546 discloses a catheter with a single curved tip.

Patent No. US2022080166 discloses a catheter with a curved tip, through which an additional element ending with a balloon is inserted, the inflation of which is to immobilize the catheter in the vessel, giving a stable position for insertion of a guidewire.

Patent No. US2022168041 discloses a catheter with an adjustable tip curvature where a system of tendons in the side walls is used, the pulling of which causes the tip of the catheter to bend.

Endovascular catheters are used as tools that allow the insertion of medical instruments into the patient's body through vascular access (most often puncture or incision of the femoral artery) through which the place where the disease occurs is reached. A guidewire is inserted through the catheter and passed through the vessels, and the curvature of the catheter tip allows the guidewire to be guided laterally, for example into a branch of a vessel. This kind of the catheter structure hinders the efficient operation of a surgeon during the operation.

Solutions that are known have one specific curvature or shape, which does not allow the operator to fully manipulate the shape, but only to move within the range allowed by the catheter and the guidewire inserted into it.

The solution according to the invention overcomes the disadvantages of the prior art. The solution according to the invention allows the surgeon to manipulate the two curves of the tubes by their mutual rotation around each other, affecting the degree of curvature of both elements, and thus easier targeting the tip of the inserted guidewire at the desired place. This shortens the operation time, facilitates the work of the surgeon and increases the patient's safety. The solution according to the invention also allows to reduce the cost of the surgical procedure by using one catheter in the subject matter solution instead of several others. The endovascular catheter according to the invention is easy to insert into the vessel. This solution enables easier insertion of the guidewire into the catheter than in the solutions hitherto known, as well as easier manipulation of the catheter tips. The use of two coaxial tubes in the catheter according to the invention is a great innovation in the treatment of vascular diseases by the intravascular method. It allows for faster and easier handling of this type of diseases, which translates into benefits for patients and the hospital. The use of the endovascular catheter according to the invention improves the safety of surgical interventions by reducing the risk of postoperative complications that may result from prolonged procedure time.

The essence of the invention is that the catheter has a two-element structure, consisting of two thin tubes of different diameters, the inner tube being thinner than the outer tube and placed inside a wider outer tube, both tubes having curved ends in the distal part, whose bend radius differs; the bend radius of the ends of both tubes in the distal section is between 45-90°; the curved ends rotate independently of each other around a common axis and longitudinally slide one tube into the other.

The endovascular catheter according to the invention is in the form of two thin tubes made of a soft material, preferably plastic. The thinner tube is placed inside the wider one and both have curved ends, but the bend radius in both differs. The material from which the catheter is made makes it possible to observe the catheters inserted into the patient's body through X-ray imaging. The catheter according to the invention has a structure that allows for separate manipulation of each tube so that the operator can manipulate the curved ends of the catheters, rotating them independently of each other around a common axis and longitudinally inserting one catheter into the other. Rotations allow the operator to change the shape of the tip from a "C"-shaped to an "S-shaped curve, and insertion allows the surgeon to change the curvature radius. The endovascular catheter according to the invention enables the manipulation of two curvatures by their mutual rotation around each other, affecting the degree of curvature of both elements and thus easier targeting the tip of the inserted guidewire at the desired location. The solution according to the invention consists in a two-piece catheter structure where the outer tube and the inner tube have curved tips and an appropriate curvature of the tip is achieved by inserting the inner tube into the outer tube. As a result, the bending angle of the tube from 45° to 90° can be achieved. The device, according to the invention, can also be used where partial insertion angles range is from 45° to 90°. In the solution according to the invention, it is also possible to rotate the inner tube along its axis, which allows to obtain almost any shape of the tip - from the shape of an arc to the shape close to the letter "S". The proximal part and the shaft in the catheter are made in a manner known in the art. The proximal part may be with a single-entry end, or be branched, with several entries. The guidewire entry must have a proximal valve. The interior of the catheter shaft is a pass-through hole and connected to the holes located around the perimeter of the catheter. The diameter of the outer (wider) tube in the catheter according to the invention is known in the art.

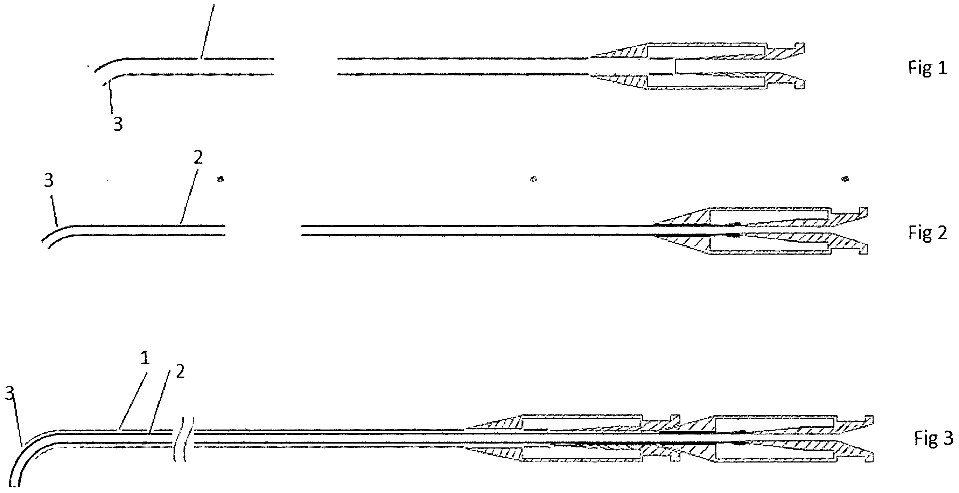

The solution according to the invention is shown in the attached drawing, figs 1-3, where:
fig. 1 shows the outer tube,
fig. 2 shows the inner tube,
fig. 3 shows the catheter folded up.

The solution according to the invention is presented in non-limiting examples of claims.

### Example 1:

The endovascular catheter according to the invention consists of a proximal part constructed in a manner known in the art. The central part of the catheter is a shaft constructed in the manner known in the art. The distal part of the catheter has curved tips (3). The endovascular catheter according to the invention consists of two detachable elements, i.e. two tubes of different diameters. The diameter of the wider outer tube (1) is known in the art. The diameter of the inner tube (2) (thinner) is slightly smaller than the diameter of the outer tube (1), so that the inner tube (2) slides into the outer tube (1) and rotates along its axis without any problems. Both tubes (1) and (2) have curved ends (3), the bending radius of which is 45 - 90°. The tubes are made of soft plastic. As the diameter of the two tubes differs by less than half a millimeter, it is possible to insert (place) the inner tube (2) into the outer tube (1) and manipulate each tube. Thereby, the curved tips (3) rotate independently of each other around a common axis and longitudinally, inserting one catheter into the other.

## Claims

1. Endovascular catheter having a proximal portion, a shaft, and a distal portion with an opening or holes, is **characterized in that** has a two-element structure, consisting of two thin tubes (1) and (2) of different diameters, the inner tube (2) being thinner than the outer tube (1) and placed inside a wider outer tube (1), both tubes (1) and (2) having curved ends (3) in the distal part, whose bend radius differs.

2. Endovascular catheter according to claim 1, is **characterized in that** the bend radius of the ends (3) of both tubes (1) and (2) in the distal section is between 45-90°; the curved ends (3) rotate independently of each other around a common axis and longitudinally slide one tube into the other.

## Amended claims

### Amended claims in accordance with Rule 137(2) EPC.

1. Endovascular catheter having a proximal portion, a shaft, and a distal portion with an opening or holes and has a two-element structure, is **characterized in that**, consisting of two thin tubes (1) and (2) of different diameters, the inner tube (2) being thinner than the outer tube (1) and placed inside a wider outer tube (1), both tubes (1) and (2) having curved ends (3) in the distal part, whose bend radius differs.

2. Endovascular catheter according to claim 1, is **characterized in that** the curved ends (3) rotate independently of each other around a common axis and longitudinally slide one tube into the other so that follows to change the shape of the tip from a "C"-shaped to an "S-shaped curve, and insertion allows to change the curvature radius
